# EUROPEAN PATENT SPECIFICATION

(11) **EP 3 955 983 B1**
(45) Date of publication and mention of the grant of the patent: **11.09.2024**
(21) Application number: 20716957.4
(22) Date of filing: 07.04.2020
(51) Int. Cl.: A61M 1/02, A61J 1/10, A61J 1/14, B67B 7/92

(54) **BREAKING DEVICE FOR BREAKING A CLOSING ELEMENT OF A BLOOD BAG**
BRECHVORRICHTUNG ZUM BRECHEN EINES VERSCHLUSSELEMENTS EINES BLUTBEUTELS
DISPOSITIF DE RUPTURE POUR ROMPRE UN ÉLÉMENT DE FERMETURE D'UN SAC DE SANG

(30) Priority: 15.04.2019 IT 201900005772
(43) Date of publication of application: 23.02.2022
(73) Proprietor: Delcon S.r.l., 24050 Grassobbio (BG) (IT)
(72) Inventor: SCACCABAROZZI, Luca, 20882 Bellusco (MB) (IT)
(74) Representative: Dragotti & Associati S.R.L.
(86) International application number: PCT/IB2020/053312
(87) International publication number: WO 2020/212803

(56) References cited:
- WO-A1-2015/113701
- IT-A1- MI20 122 028
- US-A1- 2010 132 512

## Description

### TECHNICAL FIELD

The present invention relates to the field of the devices for treating medical containers containing blood components, and in particular to a breaking device for breaking a closing element of a blood bag.

### PRIOR ART

Medical containers for liquids, for example blood bags, comprise one or more cannulas which allow the blood or blood components to exit from the container. The cannulas are normally closed by a valve or frangible septum, which is to be broken to allow the blood to exit from therein, for example when the bag is associated with a machine for extracting blood components.

Several devices capable of automatically breaking the frangible septum exist, so as to avoid such an operation to be carried out manually by an operator.

One of the main drawbacks of the automatic breaking devices, called "breakers", is due to the fact that different manufacturers make blood bags with different characteristics, not only in terms of overall dimensions of the bag and the diameter of the cannula, but also in terms of relative position of the frangible septum along the extension of the cannula.

A device for breaking cannulas of blood bags is known for example from EP2925380 to the same Applicant, the bag comprising a breaking head and replaceable cannula positioning and breaking means. According to the type of bag and the positioning of the frangible septum in the cannula, it is possible to change and replace the positioning and breaking means so as to be certain that the breaking head is always aligned with the frangible septum.

The increased use of blood components extractors in hospitals, transfusion centres and laboratories has underlined the need to speed up the cannula breaking operations of different bags as much as possible.

Moreover, the constant arising of new and different types of bags, each with different size characteristics, has highlighted the need to reduce the response times in the adaptation of a machine for extracting blood components with respect to a new type of bag.

It is thus an object of the present invention to meet such need by providing a breaking device for breaking a closing element of a blood bag which is capable of quickly and accurately breaking the frangible septum on any type of blood bag and of limiting the intervention of an operator to the greatest extent possible.

It is a further object of the present invention to provide a device capable of quickly adapting to new types of bags without the need to modify any components thereof.

Such objects are achieved by the technical features of the invention defined in independent claims 1,8,9. The dependent claims outline preferred and/or particularly advantageous aspects of the invention.

Document WO2015/113701 is part of the prior art.

### DESCRIPTION OF THE INVENTION

According to the aforesaid objects, the present invention makes available a breaking device for breaking a closing element of a medical container, comprising:
- an operating arrangement comprising an operating device and an operating element connected to the operating device,
- a breaking element connected to the operating element and particularly adapted to break the closing element of the medical container, wherein the breaking device further comprises:
   - a support plate,
   - a housing arranged movable with respect to the support plate according to a predetermined axial direction, the operating element being arranged in the housing, and
   - a first actuator arrangement connected to the support plate and interacting with the housing to selectively move the housing along said predetermined axial direction so as to selectively position the breaking element in a breaking position with the closing element of the medical container.

Thanks to such solution, it is possible to quickly adapt the breaking device to any type of blood bag, regardless of the relative position of the frangible septum inside the cannula of the bag, without replacing or modifying the breaking device.

Another aspect of the present invention provides for the breaking device to comprise a vertically extending sliding guide element coupled to the housing.

Thanks to such solution, it is possible to move the housing of the breaking element along a vertical axial direction, avoiding possible misalignments and strengthening, in use, the resistance to lateral stresses due to the movement of the breaking element.

Another aspect of the present invention provides for the sliding guide to comprise at least one guide pin inserted protruding in a through hole made on the support plate, a vertically extending tubular structure being engaged with said pin.

Another aspect of the present invention provides for the actuator arrangement to comprise a threaded screw inserted in a through hole made in a side portion of the plate and in a through hole made in the housing, the through hole comprising a threaded surface therein. Thanks to such solution, it is possible to make a screw-leadscrew coupling which allows a quick and easy adjustment of the position of the breaking element.

Another aspect of the present invention provides for the operating device to comprise a drive shaft, the operating element to comprise a shaft connected to the drive shaft, the axis of the shaft being arranged parallel to the axis of the drive shaft.

Thanks to such solution, it is possible to significantly reduce the overall dimensions of the breaking device in a blood components extractor.

A further aspect of the present invention provides for the operating device to comprise a belt and a pair of pulleys respectively keyed onto the drive shaft and onto the shaft. Another aspect again of the present invention provides for the operating device to comprise a drive shaft, the operating element to comprise a shaft connected to the drive shaft, the shaft being arranged in alignment with the drive shaft.

Thanks to such solution, it is possible to reduce the number of components of the breaking device, thus reducing the production costs and facilitating the maintenance thereof.

A further aspect of the present invention provides for the breaking device to comprise a second operating arrangement for selectively operating the first actuator arrangement. Thanks to such solution, it is possible to avoid the operator from manually acting on the adjustment of the position of the breaking element, thus further speeding up the times and avoiding possible positioning errors.

The present invention also makes available a machine for extracting blood components from a medical container comprising a closing element, characterized in that it comprises a breaking device as defined above.

The present invention also makes available a method for breaking a closing element of a medical container, comprising the steps of:
- providing an operating arrangement comprising an operating device and an operating element connected to the operating device,
- providing a breaking element connected to the operating element and particularly adapted to break the closing element of the medical container, characterized in that it further comprises the steps of:
- selectively positioning the breaking element in a breaking position with the closing element of the medical container, thus modifying the position of the breaking element along a predetermined axial direction.

### BRIEF DESCRIPTION OF THE DRAWINGS

Further features and advantages of the present invention will become apparent from the following description, given only by way of example, with reference to the accompanying drawings, in which:
- figure 1 is a partial perspective view of a machine for extracting blood components, in a first operating position;
- figure 2 is a partial perspective view of the machine in figure 1, in a second operating position;
- figure 3 is a perspective view of a breaking device according to the present invention;
- figure 4 is a plan view of the device in figure 2;
- figure 5 is a sectional view of the breaking device along line V-V in figure 4;
- figure 6 is a sectional view of the breaking device along line VI-VI in figure 4;
- figure 7 is a perspective view of a further embodiment of a breaking device according to the present invention;
- figure 8 is a sectional view of the device in figure 7;
- figure 9 is a perspective view of a further embodiment of a breaking device according to the present invention; and
- figures 10 and 11 are sectional views of the device in figure 9.

Identical reference numbers have been used when possible to identify common identical elements in the drawings to facilitate comprehension. It is to be understood that elements and characteristics of one embodiment may conveniently be incorporated into other embodiments without further clarifications.

### IMPROVED METHOD FOR IMPLEMENTING THE INVENTION

Reference is now made in detail to various embodiments of the invention, one or more of which are shown in the accompanying drawings. Each example is provided merely to illustrate the invention and is not intended as a limitation thereof. For example, the technical characteristics illustrated or described because they belong to one embodiment may be implemented in, or in association with, other embodiments in order to generate a further embodiment. It is understood that the present invention is comprehensive of such modifications and variants.

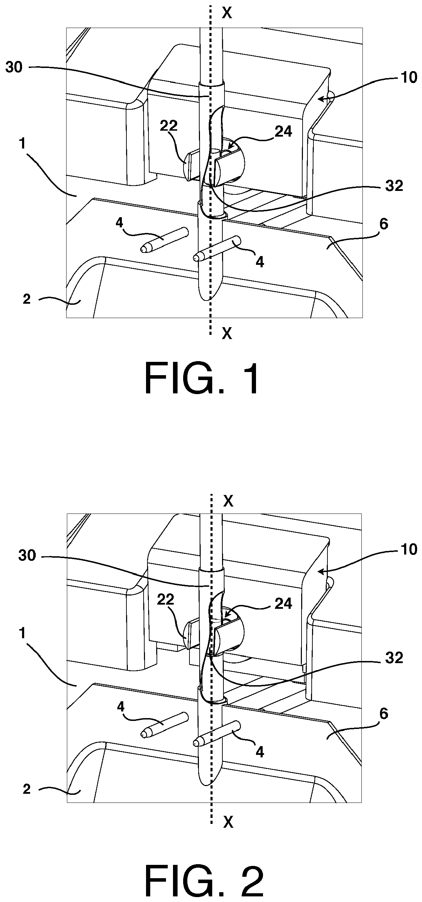

Figure 1 illustrates a partial perspective view of a machine 1 for extracting blood components, i.e. a blood components extractor, and in particular of a breaking device 10 for breaking a closing element of a blood bag 2 associated with said machine 1.

The machine 1 for extracting blood components comprises a hanging area where the bag is hung to then be processed. In particular, the hanging area comprises a pair of pins 4 which are particularly adapted to receive a pair of through holes made on an end, for example the upper end 6, of a blood bag 2. The blood bag 2 comprises at least one cannula 30 which extends along a predetermined axial direction X-X.

The breaking device 10 is arranged at the hanging area 12 and comprises a breaking element 20, which in the embodiment illustrated in figure 1, has a "U"- shaped cross section, consisting of a pair of parallel arms 22 defining a housing area 24 therein.

In use, a portion of the cannula 30 is positioned in the housing area 24, and in particular the portion of a cannula 30 comprising a closing element, for example a frangible septum 32, in such a manner that the interaction of the breaking element 20 with the closing element 32 allows the breaking of the latter, as is more apparent below.

Naturally, it is possible to provide different embodiments of the breaking element 20, also of known type, without departing from the scope of the present invention as long as said breaking element is capable of breaking the frangible septum 32 of a cannula 30.

The breaking device 10 according to the present invention comprises at least one operating arrangement comprising an operating device 50, a motor by way of non-limiting example, and an operating element 60 operatively connected, in use, to the motor 50.

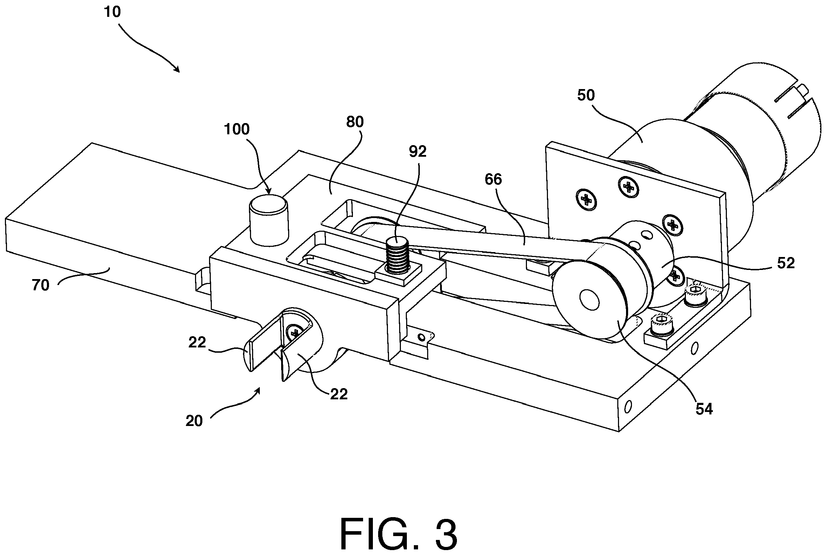

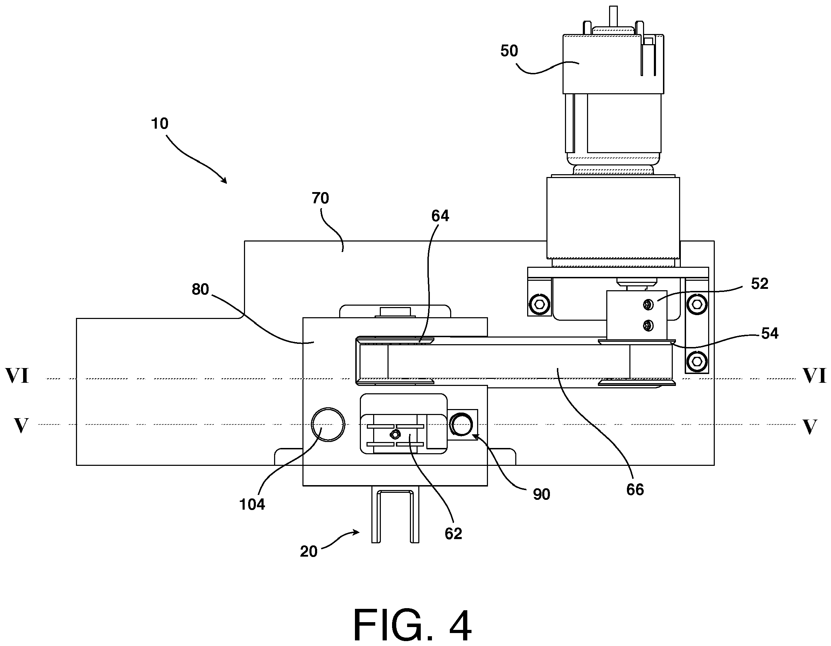

In one of the possible embodiments illustrated in figures 3 to 6, the motor 50 comprises a drive shaft 52, onto one end of which a toothed wheel 54, or pulley, is keyed. The operating element 60 comprises a shaft 62 onto which first end a toothed wheel 64, or pulley, is keyed, and with which second end the breaking element 20 is engaged. According to this configuration, the axis of the shaft 62 is arranged parallel to the axis of the drive shaft 52. The operating arrangement further comprises a belt 66 or other mechanical drive member.

The drive shaft 52 is operatively connected to the shaft 62 by means of the belt 66 in such a manner as to be placed, in use, into rotation by the motor 50.

The breaking device 10 comprises a support plate 70 which is particularly adapted to be fixed integral with a frame of the machine 1 for extracting blood components. According to alternative embodiments of the present invention, the support plate 70 is part of the frame of the machine 1 for extracting blood components.

The breaking device 10 further comprises a housing 80, arranged movable on the support plate 70. The shaft 62 of the operating arrangement is arranged in the housing 80.

The breaking device 10 comprises an actuator arrangement 90 connected to the support plate 70 and to the housing 80, which is particularly adapted to selectively move, in use, the housing 80 to or from the support plate 70 in order to selectively position the breaking element 20 in an engagement position with the closing element 32 of the cannula 30, i.e. to adjust the relative position of the breaking element 20 with respect to the closing element 32.

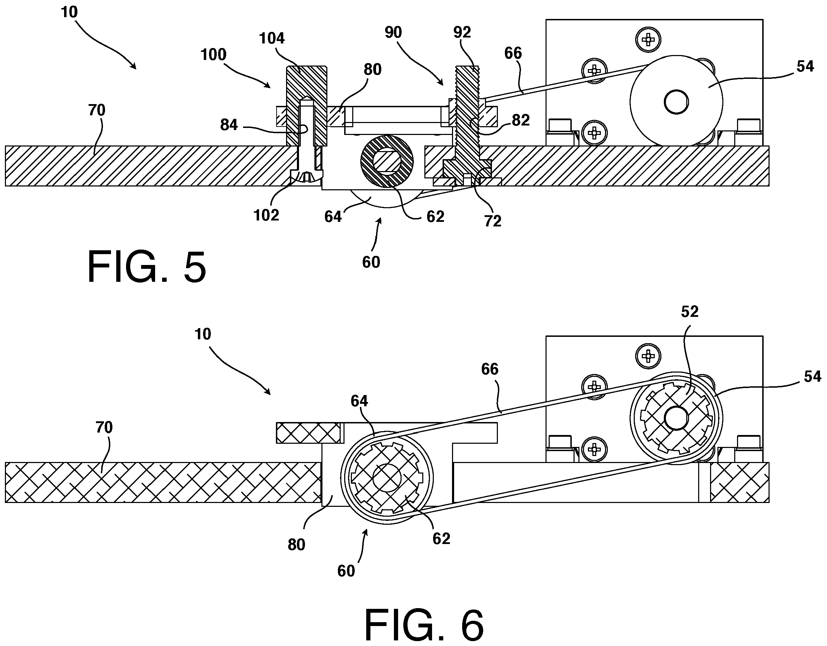

As better illustrated in figure 5, according to one of the possible embodiments of the present invention, the actuator arrangement 90 comprises a threaded screw 92 inserted in a through hole 72 made in a side portion of the plate 70 and close to the breaking head 20, and in a through hole 82 made in the housing 80. The through hole 82 comprises a threaded surface therein having the same characteristics as the threading of the screw 92 in such a manner as to form a screw-leadscrew coupling together with the threaded screw 92.

Naturally, the embodiments herein described and illustrated are only one of the possible embodiments of an actuator arrangement capable of selectively positioning the breaking element 20 in an engagement position with the closing element 32 of the cannula 30. For example, it is possible to provide for hydraulic, electric, electromagnetic and/or electrostatic actuators, operated manually or by means of further operating arrangements, as described below.

According to a further embodiment of the present invention, the breaking device 10 comprises a vertically extending sliding guide 100, i.e. according to the predetermined axial direction X-X, coupled to the housing 80.

In the embodiment illustrated in figures 3 to 6, the sliding guide 100 comprises a guide pin 102 inserted protruding in a through hole made on the support plate 70. A sleeve 104, or other vertically extending tubular structure, is engaged with the pin 102.

The housing 80 is slidably constrained to the sleeve 104 of the guide pin 102 by means of a second through hole 84.

Naturally, the embodiments herein described and illustrated are only one of the possible embodiments of a sliding guide capable of promoting the translation of the breaking element 20 along a predetermined axial direction.

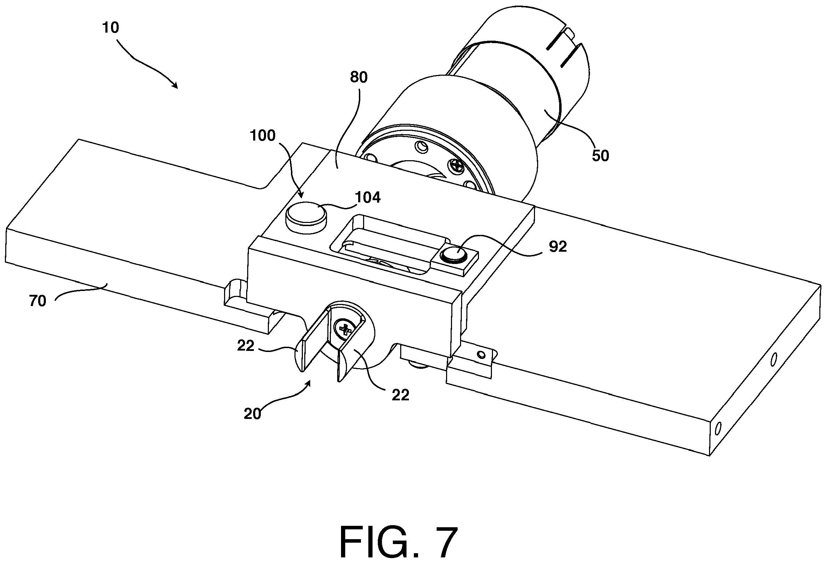

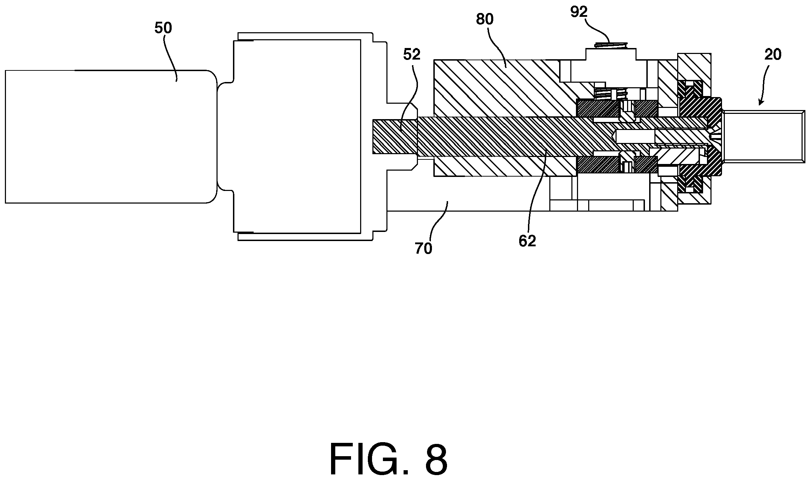

A further one of the embodiments of the present invention illustrated in figures 7 and 8 provides for an operating arrangement comprising an operating device 50, a motor by way of non-limiting example, and an operating element 60 operatively connected, in use, to the motor 50.

The motor 50 comprises a drive shaft 52 and the operating element 60 comprises a shaft 62 directly engaged with a first end thereof with the drive shaft 52. The second end of the shaft 62 is engaged with the breaking element 20. According to this configuration, the shaft 62 is arranged in alignment with the drive shaft 52.

Here too, the actuator arrangement 90 comprises a threaded screw 92 inserted in a through hole made in a middle portion of the plate 70 and in a through hole made in the housing 80. The through hole comprises a threading therein having the same characteristics as the threading of the screw 92 in such a manner as to form a screw-leadscrew coupling together with the threaded screw 92.

Similarly, the sliding guide 100 comprises a guide pin inserted protruding in a through hole made on the support plate 70. A sleeve 104, or other vertically extending tubular structure, is engaged with the guide pin.

The housing 80 is slidably constrained to the sleeve 104 of the guide pin by means of a second through hole.

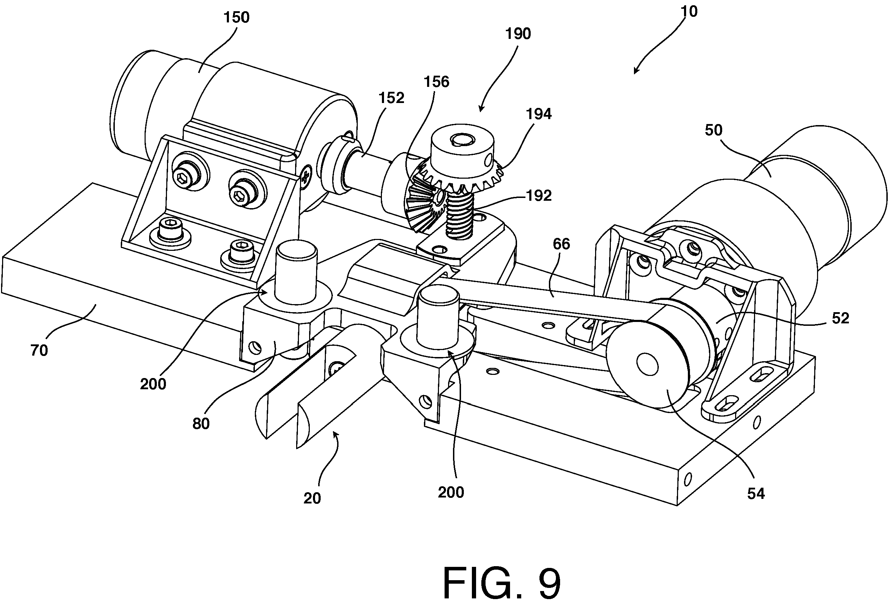

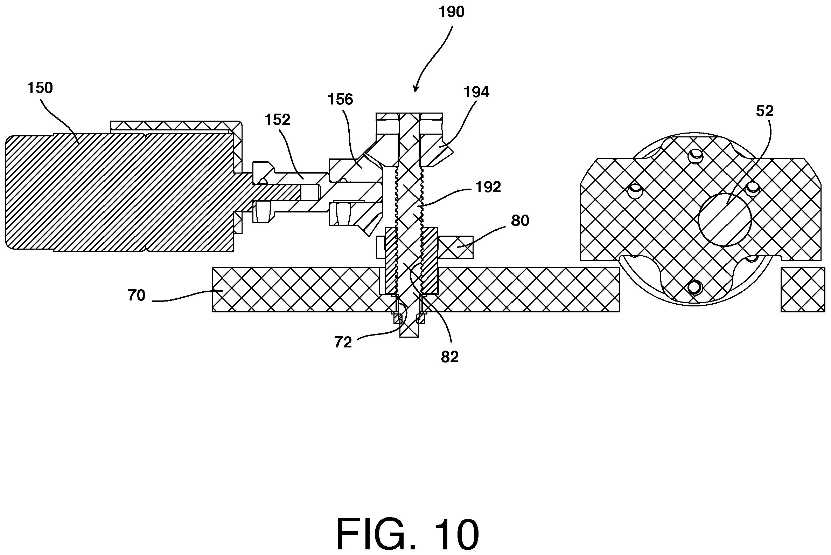

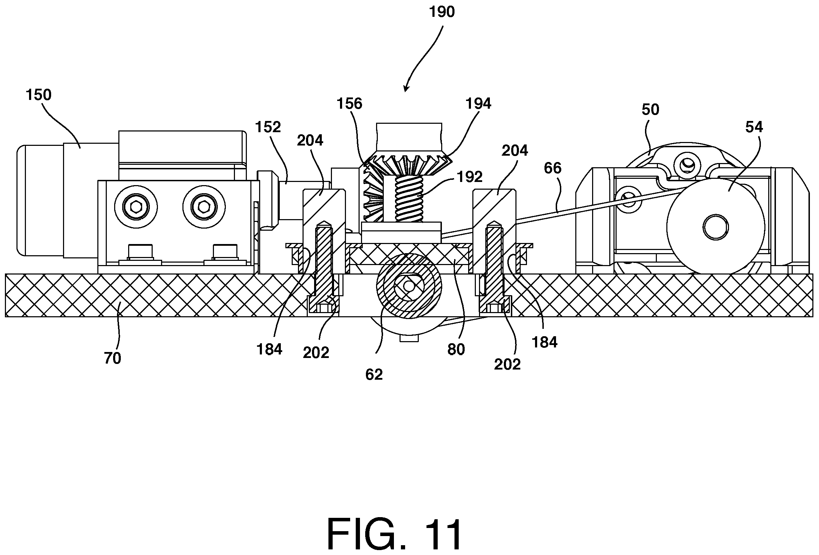

With reference now to figures 9 to 11, according to a further one of the embodiments of the present invention, the breaking device 10 comprises a first operating arrangement comprising an operating device 50, a motor by way of non-limiting example, and an operating element 60 operatively connected, in use, to the motor 50.

As described above with reference to figures 3 to 6, in one of the possible embodiments of the present invention, the motor 50 comprises a drive shaft 52 operatively connected to a shaft 62 of the operating element 60 by means of a belt 66 and a pair of pulleys 54, 64 respectively keyed onto the drive shaft 53 and onto the shaft 62. The breaking element 20 is engaged at an end of the shaft 62.

The breaking device 10 may comprise a support plate 70 which is particularly adapted to be fixed integral with a frame of the machine 1 for extracting blood components, and a housing 80, arranged movable on the support plate 70. The shaft 62 is arranged in the housing 80. According to alternative embodiments of the present invention, the support plate 70 is part of the frame of the machine 1 for extracting blood components.

The breaking device 10 comprises an actuator arrangement 190 connected to the support plate 70, which is particularly adapted to selectively move, in use, the housing 80 to or from the support plate 70 in order to selectively position the breaking element 20 in an engagement position with the closing element 32 of the cannula 30, i.e. to adjust the relative position of the breaking element 20 with respect to the closing element 32. According to this embodiment, the actuator arrangement 190 comprises a threaded screw 192 inserted in a through hole 72 made in a middle portion of the plate 70 but arranged opposite to the end of the shaft 62 connected to the breaking head 2, and in a through hole 82 made in the housing 80. The through hole 82 comprises a threading therein having the same characteristics as the threading of the screw 192 in such a manner as to form a screw-leadscrew coupling together with the threaded screw 192.

A conical toothed wheel 194 is fixed on the upper portion of the threaded screw 192.

The breaking device 10 comprises a second operating arrangement, comprising a motor 150 and a drive shaft 152. A second conical toothed wheel 156 is fixed on the outer end of the drive shaft 152, which wheel is engaged with the first toothed wheel 194 to transmit, in use, a rotating movement to the threaded screw 192 of the actuator arrangement 190. Here too, the embodiments herein described and illustrated are only one of the possible embodiments of the second actuator arrangement capable of selectively operating the first actuator arrangement to bring the breaking element 20 in an engagement position with the closing element 32 of the cannula 30. For example, it is possible to provide different hydraulic, electric, electromagnetic and/or electrostatic actuators.

The breaking device 10 further comprises a vertically extending sliding guide 200 coupled to the housing 80.

In the embodiment illustrated in figures 9 to 11, the sliding guide 100 comprises two guide pins 202 each inserted protruding in a through hole made on the support plate 70. A sleeve 204, or other vertically extending tubular structure, is engaged with each pin 202. According to this configuration, the housing 80 is slidably constrained to the sleeves 204 of the guide pins 202 by means of a pair of through holes 184 made on the two corner ends of the housing 80 in the portion arranged close to the shaft 62 connected to the breaking head 20.

In use, a bag 2 is hung on a blood components extractor 1, and in particular on the pins 4. A cannula 30 of the bag 2 is inserted in the housing area 24 of the breaking element 20. Then, the operating arrangement is actuated, and in particular the motor 50 is actuated, providing the drive shaft 52 with a reciprocating rotating motion according to a predetermined cycle. The drive shaft 52 transmits the reciprocating rotating motion to the shaft 62 by means of the pulleys 54, 64 and the belt 66. The shaft 62 carries the breaking head 20 to rotate according to a reciprocating rotating motion in two opposite directions, thus folding the portion of cannula 30 contained in the housing 24 according to two opposite directions until the frangible septum 32 breaks due to the transverse stresses received.

Once the frangible septum 32 has broken, the blood components extractor 1 completes the operating cycle thereof and the bag 2 is replaced with a new bag 2.

If the new bag 2 comprises a cannula 30 in which the frangible septum 32 is arranged in the same relative position as the bag 1, the above steps are repeated.

If the new bag 2 comprises a cannula 30 in which the frangible septum 32 is arranged along the predetermined axial direction X-X in a different relative position with respect to the preceding bag, and if the frangible septum 32 is no longer arranged in the housing 24, it is necessary to modify the position of the breaking element 20 along the predetermined axial direction X-X prior to activating the operating arrangement so that the housing 24 is arranged at the frangible septum 32.

According to the embodiments illustrated in figures 3 to 8, the operator activates the actuator arrangement 90 by bringing the threaded screw 92 into rotation. The rotation direction of the threaded screw 92 depends on the position of the frangible septum with respect to the breaking element 20. A first rotation direction brings the housing 80 towards the plate 70, and therefore lower with respect to the machine 1, along the predetermined axial direction X-X. A second rotation direction brings the housing 80 away from the plate 70, and therefore higher with respect to the machine 1, along the predetermined axial direction X-X, as illustrated in figure 2.

Once the housing 24 is arranged at the frangible septum 32, i.e. the breaking element 20 is aligned with the frangible septum, the operating arrangement is activated to allow the breaking of the frangible septum 32, as described above.

According to the embodiment illustrated in figures 9 to 11, if the frangible septum 32 of a bag 2 is not arranged in the housing 24, the position of the breaking element 20 is modified, activating the second operating arrangement, and in particular the motor 150. A rotation of the drive shaft 152 brings into rotation the conical toothed wheel 156, which in turn drags into rotation the conical toothed wheel 194, which allows the rotation of the threaded screw 192.

The rotation direction of the threaded screw 192, and therefore of the drive shaft 152, depends on the position of the frangible septum with respect to the breaking element 20. A first rotation direction brings the housing 80 towards the plate 70, and therefore lower with respect to the machine 1, along the predetermined axial direction X-X. A second rotation direction brings the housing 80 away from the plate 70, and therefore higher with respect to the machine 1, along the predetermined axial direction X-X, as illustrated in figure 2.

Once the housing 24 is arranged at the frangible septum 32, i.e. the breaking element 20 is aligned with the frangible septum, the operating arrangement is activated to allow the breaking of the frangible septum 32, as described above.

Each time a bag 2 with different characteristics, i.e. each time a bag with a frangible septum 32 arranged in a different relative position in the cannula 30 along the predetermined axial direction X-X, is hung on the machine 1, it is possible to modify the position of the breaking element 20 as described above, so as to align the breaking element 20 with the frangible septum 32, without modifying the breaking device 1 and without replacing the device or one or more parts thereof.

## Claims

1. A breaking device for breaking a closing element of a medical container, comprising:
- an operating arrangement comprising an operating device (50) and an operating element (60) connected to the operating device (50),
- a breaking element (20) connected to the operating element (60) and particularly adapted to break the closing element (32) of the medical container (2),
wherein the breaking device (10) further comprises:
- a support plate (70),
- a housing (80) arranged movable with respect to the support plate (70) according to a predetermined axial direction (X-X), the operating element (60) being arranged in the housing (80), and
- a first actuator arrangement (90) connected to the support plate (70) and interacting with the housing (80) to selectively move the housing (80) along said predetermined axial direction (X-X) so as to selectively position the breaking element (20) in a breaking position with the closing element (32) of the medical container (2).

2. Breaking device according to claim 1, **characterized in that** it further comprises a vertically extending sliding guide element (100, 200) coupled to the housing (80).

3. Breaking device according to claim 2, **characterized in that** the sliding guide (100) comprises at least one guide pin (102, 202) inserted protruding in a through hole made on the support plate (70), a vertically extending tubular structure (104, 204) being engaged with said pin (102, 202).

4. Breaking device according to any one of the preceding claims, **characterized in that** the actuator arrangement (90) comprises a threaded screw (92) inserted in a through hole (72) made in a side portion of the plate (70) and in a through hole (82) made in the housing (80), the through hole (72) comprising a threaded surface therein.

5. Breaking device according to any one of the preceding claims, **characterized in that** the operating device (50) comprises a drive shaft (52), the operating element (60) comprises a shaft (62) connected to the drive shaft (50), the axis of the shaft (62) being arranged parallel to the axis of the drive shaft (52).

6. Breaking device according to claim 5, **characterized in that** the operating device (50) comprises a belt (66) and a pair of pulleys (54, 64) respectively keyed onto the drive shaft (53) and onto the shaft (62).

7. Breaking device according to any one of claims 1 to 4, **characterized in that** the operating device (50) comprises a drive shaft (52), the operating element (60) comprises a shaft (62) connected to the drive shaft (50), the shaft (62) being arranged parallel in alignment with the drive shaft (52).

8. Breaking device according to any one of the preceding claims, **characterized in that** the breaking device (10) comprises a second operating arrangement (150, 152) for selectively operating the first actuator arrangement (190).

9. Machine for extracting blood components from a medical container comprising a closing element and a breaking device as defined in any one of claims 1 to 8.

10. Method for breaking a closing element of a medical container, comprising the steps of:
- providing an operating arrangement comprising an operating device (50) and an operating element (60) connected to the operating device (50),
- providing a breaking element (20) connected to the operating element (60) and particularly adapted to break the closing element (32) of the medical container (2),
**characterized in that** it further comprises the steps of:
- selectively positioning the breaking element (20) in a breaking position with the closing element (32) of the medical container (2), thus modifying the position of the breaking element (20) along a predetermined axial direction (X-X).

## Patentansprüche

1. Brechvorrichtung zum Brechen eines Verschlusselements eines medizinischen Behälters, umfassend:
- eine Bedienanordnung, die eine Bedienvorrichtung (50) und ein mit der Bedienvorrichtung (50) verbundenes Bedienelement (60) umfasst,
- ein Brechelement (20), das mit dem Bedienelement (60) verbunden und insbesondere dazu ausgelegt ist, das Verschlusselement (32) des medizinischen Behälters (2) zu brechen,
wobei die Brechvorrichtung (10) weiter Folgendes umfasst:
- eine Trägerplatte (70),
- ein Gehäuse (80), das in Bezug auf die Trägerplatte (70) gemäß einer vorgegebenen axialen Richtung (X-X) beweglich angeordnet ist, wobei das Bedienelement (60) in dem Gehäuse (80) angeordnet ist, und
- eine erste Aktuatoranordnung (90), die mit der Trägerplatte (70) verbunden ist und mit dem Gehäuse (80) zusammenwirkt, um das Gehäuse (80) selektiv entlang der vorbestimmten axialen Richtung (X-X) zu bewegen, um das Brechelement (20) selektiv in einer Brechposition mit dem Verschlusselement (32) des medizinischen Behälters (2) zu positionieren.

2. Brechvorrichtung nach Anspruch 1, **dadurch gekennzeichnet, dass** sie weiter ein vertikal verlaufendes Gleitführungselement (100, 200) umfasst, das mit dem Gehäuse (80) gekoppelt ist.

3. Brechvorrichtung nach Anspruch 2, **dadurch gekennzeichnet, dass** die Gleitführung (100) mindestens einen Führungsstift (102, 202) umfasst, der in ein in der Trägerplatte (70) ausgebildetes Durchgangsloch eingesetzt ist, wobei eine sich vertikal verlaufende rohrförmige Struktur (104, 204) mit dem Stift (102, 202) in Eingriff steht.

4. Brechvorrichtung nach einem der vorstehenden Ansprüche, **dadurch gekennzeichnet, dass** die Aktuatoranordnung (90) eine Gewindeschraube (92) umfasst, die in ein in einem Seitenabschnitt der Platte (70) ausgebildetes Durchgangsloch (72) und in ein im Gehäuse (80) ausgebildetes Durchgangsloch (82) eingesetzt ist, wobei das Durchgangsloch (72) eine Gewindefläche umfasst.

5. Brechvorrichtung nach einem der vorstehenden Ansprüche, **dadurch gekennzeichnet, dass** die Bedienvorrichtung (50) eine Antriebswelle (52) umfasst, das Bedienelement (60) eine mit der Antriebswelle (50) verbundene Welle (62) umfasst, wobei die Achse der Welle (62) parallel zur Achse der Antriebswelle (52) angeordnet ist.

6. Brechvorrichtung nach Anspruch 5, **dadurch gekennzeichnet, dass** die Bedienvorrichtung (50) einen Riemen (66) und ein Paar Riemenscheiben (54, 64) umfasst, die auf der Antriebswelle (53) bzw. auf der Welle (62) verkeilt sind.

7. Brechvorrichtung nach einem der Ansprüche 1 bis 4, **dadurch gekennzeichnet, dass** die Bedienvorrichtung (50) eine Antriebswelle (52) umfasst, das Bedienelement (60) eine mit der Antriebswelle (50) verbundene Welle (62) umfasst, wobei die Welle (62) parallel in Ausrichtung mit der Antriebswelle (52) angeordnet ist.

8. Brechvorrichtung nach einem der vorstehenden Ansprüche, **dadurch gekennzeichnet, dass** die Brechvorrichtung (10) eine zweite Bedienanordnung (150, 152) zum selektiven Betrieb der ersten Aktuatoranordnung (190) umfasst.

9. Maschine zur Entnahme von Blutkomponenten aus einem medizinischen Behälter, umfassend ein Verschlusselement und eine Brechvorrichtung nach einem der Ansprüche 1 bis 8.

10. Verfahren zum Aufbrechen eines Verschlusselementes eines medizinischen Behälters, umfassend die Schritte:
- Bereitstellen einer Bedienanordnung, die eine Bedienvorrichtung (50) und ein mit der Bedienvorrichtung (50) verbundenes Bedienelement (60) umfasst,
- Bereitstellen eines Brechelements (20), das mit dem Bedienelement (60) verbunden und insbesondere dazu ausgelegt ist, das Verschlusselement (32) des medizinischen Behälters (2) zu brechen, **dadurch gekennzeichnet, dass** es weiter die folgenden Schritte umfasst:
- selektives Positionieren des Brechelements (20) in einer Brechposition mit dem Verschlusselement (32) des medizinischen Behälters (2), wodurch die Position des Brechelements (20) entlang einer vorgegebenen axialen Richtung (X-X) verändert wird.

## Revendications

1. Dispositif de rupture pour rompre un élément de fermeture d'un récipient médical, comprenant :
- un agencement de commande comprenant un dispositif de commande (50) et un élément de commande (60) relié au dispositif de commande (50),
- un élément de rupture (20) relié à l'élément de commande (60) et particulièrement adapté pour rompre l'élément de fermeture (32) du récipient médical (2),
dans lequel le dispositif de rupture (10) comprend en outre :
- une plaque de support (70),
- un logement (80) agencé de manière mobile par rapport à la plaque de support (70) selon une direction axiale prédéterminée (X-X), l'élément de commande (60) étant agencé dans le logement (80), et
- un premier agencement d'actionnement (90) relié à la plaque de support (70) et interagissant avec le logement (80) pour déplacer sélectivement le logement (80) le long de ladite direction axiale prédéterminée (X-X) de manière à positionner sélectivement l'élément de rupture (20) dans une position de rupture avec l'élément de fermeture (32) du récipient médical (2).

2. Dispositif de rupture selon la revendication 1, **caractérisé en ce qu'**il comprend en outre un élément de guidage coulissant s'étendant verticalement (100, 200) couplé au logement (80).

3. Dispositif de rupture selon la revendication 2, **caractérisé en ce que** le guide coulissant (100) comprend au moins une broche de guidage (102, 202) insérée en saillie dans un trou traversant pratiqué sur la plaque de support (70), une structure tubulaire s'étendant verticalement (104, 204) étant mise en prise avec ladite broche (102, 202).

4. Dispositif de rupture selon l'une quelconque des revendications précédentes, **caractérisé en ce que** l'agencement d'actionnement (90) comprend une vis filetée (92) insérée dans un trou traversant (72) pratiqué dans une portion latérale de la plaque (70) et dans un trou traversant (82) pratiqué dans le logement (80), le trou traversant (72) comprenant une surface filetée dans celui-ci .

5. Dispositif de rupture selon l'une quelconque des revendications précédentes, **caractérisé en ce que** le dispositif de commande (50) comprend un arbre d'entraînement (52), l'élément de commande (60) comprend un arbre (62) relié à l'arbre d'entraînement (50), l'axe de l'arbre (62) étant agencé parallèlement à l'axe de l'arbre d'entraînement (52).

6. Dispositif de rupture selon la revendication 5, **caractérisé en ce que** le dispositif de commande (50) comprend une courroie (66) et une paire de poulies (54, 64) respectivement clavetées sur l'arbre d'entraînement (53) et sur l'arbre (62).

7. Dispositif de rupture selon l'une quelconque des revendications 1 à 4, **caractérisé en ce que** le dispositif de commande (50) comprend un arbre d'entraînement (52), l'élément de commande (60) comprend un arbre (62) relié à l'arbre d'entraînement (50), l'arbre (62) étant agencé parallèlement de manière alignée avec l'arbre d'entraînement (52).

8. Dispositif de rupture selon l'une quelconque des revendications précédentes, **caractérisé en ce que** le dispositif de rupture (10) comprend un second agencement de commande (150, 152) pour faire fonctionner sélectivement le premier agencement d'actionnement (190).

9. Machine pour extraire des composants sanguins d'un récipient médical comprenant un élément de fermeture et un dispositif de rupture tel que défini dans l'une quelconque des revendications 1 à 8.

10. Procédé de rupture d'un élément de fermeture d'un récipient médical, comprenant les étapes consistant à :
- fournir un agencement de commande comprenant un dispositif de commande (50) et un élément de commande (60) relié au dispositif de commande (50),
- fournir un élément de rupture (20) relié à l'élément de commande (60) et particulièrement adapté pour rompre l'élément de fermeture (32) du récipient médical (2), **caractérisé en ce qu'**il comprend en outre les étapes consistant à :
- positionner sélectivement l'élément de rupture (20) dans une position de rupture avec l'élément de fermeture (32) du récipient médical (2), modifiant ainsi la position de l'élément de rupture (20) le long d'une direction axiale prédéterminée (X-X).
